# EUROPEAN PATENT APPLICATION

(11) **EP 2 130 813 A1**
(43) Date of publication of application: **09.12.2009**
(21) Application number: 08157513.6
(22) Date of filing: 03.06.2008
(51) Int. Cl.: C07C 17/02, C07C 17/156, C07C 17/25, C07C 19/045, C07C 21/06

(54) **Process for the manufacture of at least one ethylene derivative compound**

(71) Applicant: SOLVAY (Société Anonyme), 1050 Bruxelles (BE)
(72) Inventor: The designation of the inventor has not yet been filed
(74) Representative: Jacques, Philippe

(57) **Abstract**

Process for the manufacture of at least one ethylene derivative compound starting with a hydrocarbon source according to which :
a) the hydrocarbon source is subjected to a simplified cracking which produces a mixture of products containing ethylene and other constituents ;
b) the said mixture of products is separated in one separation step into a fraction containing almost all the ethylene (fraction A) and into a heavy fraction (fraction C) ;
c) fraction A is conveyed to the manufacture of at least one ethylene derivative compound.

## Description

The present invention relates to a process for the manufacture of at least one ethylene derivative compound, in particular to a process for the manufacture of 1,2-dichloroethane (DCE) and optionally also of at least one ethylene derivative compound manufactured directly starting with ethylene which is different from DCE.

To date, ethylene which is more than 99.8 % pure is usually used for the manufacture of ethylene derivative compounds, in particular of DCE. This ethylene of very high purity is obtained via the cracking of various petroleum products, followed by numerous complex and expensive separation operations in order to isolate the ethylene from the other products of cracking and to obtain a product of very high purity.

Given the high costs linked to the production of ethylene of such high purity, various processes for the manufacture of ethylene derivative compounds, in particular DCE, using ethylene having a purity of less than 99.8 % have been developed. These processes have the advantage of reducing the costs by simplifying the course of separating the products resulting from the cracking and by thus abandoning complex separations which are of no benefit for the manufacture of ethylene derivative compounds, in particular DCE.

For example, patent application WO 00/26164 describes a process for the manufacture of DCE by simplified cracking of ethane coupled with chlorination of ethylene. To this effect, an ethylene chlorination step takes place in the presence of the impurities obtained during the cracking of the ethane.

Patent application WO 03/048088 describes the production of low-concentration ethylene for the chemical reaction with chlorine by means of ethane dehydrogenation. The ethane-loaded gas stream contains not only hydrogen and methane, but also high amounts of unconverted ethane. For the economic design of the process, the unconverted ethane must be fed back to ethane dehydrogenation after complicated cleaning processes. This process can only use ethane as feedstock. A significant disadvantage is the very low concentration of ethylene - less than 60 % - as well as the fact that further components of the gas stream such as hydrogen, propylene, butadiene only allow to use the ethylene in very special processes.

Further, patent applications WO 2006/067188, WO 2006/067190, WO 2006/067191, WO 2006/067192, WO 2006/067193 and WO 2007/147870 describe processes for the manufacture of DCE starting from a hydrocarbon source, in particular naphtha, gas oil, natural gas liquid, ethane, propane, butane, isobutane or mixtures thereof, which is first subjected to a simplified cracking. Two different factions containing ethylene are afterwards separated from the gas mixture issued from the simplified cracking before being conveyed independently to a chlorination reactor and to an oxychlorination reactor in order to produce DCE. Those processes, the aim of which is to produce and use ethylene having a purity of less than 99.8 %, present however the desadvantages of requiring several steps of separation in order to obtain the two fractions containing ethylene which complicate them and increase their costs.

Patent applications WO2008/000705, WO2008/000702 and WO2008/000693 describe, for their part, processes for the manufacture of DCE starting from a stream of ethane which is first subjected to a catalytic oxydehydrogenation. The processes described in the above-mentioned patent applications, the aim of which is to produce and use ethylene having a purity of less than 99.8 %, present however the desadvantages of requiring a first step of catalytic oxydehydrogenation which needs an important investment causing an increase in the production costs.

The aim of the present invention, for its part, is to provide a process for the manufacture of at least one ethylene derivative compound, in particular of at least DCE, using ethylene with a purity of less than 99.8 % which does not present the disadvantages of the above-mentioned processes using ethylene having a purity of less than 99.8 %.

To this effect, the invention relates to a process for the manufacture of at least one ethylene derivative compound starting with a hydrocarbon source according to which :
a) the hydrocarbon source is subjected to a simplified cracking which produces a mixture of products containing ethylene and other constituents ;
b) the said mixture of products is separated in one separation step into a fraction containing almost all the ethylene (fraction A) and into a heavy fraction (fraction C) ;
c) fraction A is conveyed to the manufacture of at least one ethylene derivative compound.

The expression "at least one ethylene derivative compound" is understood to mean, for the purpose of the present invention, that one or more than one ethylene derivative compounds may be manufactured by the process according to the present invention.

The expression "ethylene derivative compound", used hereafter in the singular or in the plural, is understood to mean, for the purpose of the present invention, any ethylene derivative compound manufactured directly starting with ethylene as well as any compound derived there from.

The expression "ethylene derivative compound manufactured directly starting with ethylene", used hereafter in the singular or in the plural, is understood to mean, for the purpose of the present invention, any compound manufactured directly from ethylene.

The expression "compound derived there from", used hereafter in the singular or in the plural, is understood to mean, for the purpose of the present invention, any compound manufactured from one compound itself manufactured from ethylene as well as any compound derived there from.

As examples of such ethylene derivative compounds manufactured directly starting with ethylene, may be cited among others, ethylene oxide, linear alpha-olefines, linear primary alcohols, homopolymers and copolymers of ethylene, ethylbenzene, vinyl acetate, acetaldehyde, ethyl alcohol, propionaldehyde and DCE.

As examples of such compound derived there from, may be cited among others,
- glycols and ethers manufactured from ethylene oxide,
- styrene manufactured from ethylbenzene and polymers of styrene derived from styrene,
- VC manufactured from DCE,
- vinylidene chloride, fluorinated hydrocarbons and PVC derived from VC and fluorinated polymers derived from fluorinated hydrocarbons, as well as
- polyvinylidene chloride and fluorinated hydrocarbons (and fluorinated polymers) derived from vinylidene chloride.

The process according to the invention is a process starting with a hydrocarbon source.

The hydrocarbon source considered may be any known hydrocarbon source. Preferably, the hydrocarbon source subjected to cracking (step a)) is chosen from the group consisting of naphtha, gas oil, natural gas liquid, ethane, propane, butane, isobutane and mixtures thereof. In a particularly preferred manner, the hydrocarbon source is chosen from the group consisting of ethane, propane, butane and propane/butane mixtures. In a more particularly preferred manner, the hydrocarbon source is chosen from the group consisting of propane, butane and propane/butane mixtures. The propane/butane mixtures may exist as such or may consist of mixtures of propane and butane.

The expression ethane, propane, butane and propane/butane mixtures is understood to mean, for the purposes of the present invention, products that are commercially available, namely that consist mainly of the pure product (ethane, propane, butane or propane/butane as a mixture) and secondarily of other saturated or unsaturated hydrocarbons, which are lighter or heavier than the pure product itself.

In the process for the manufacture of DCE and of at least one ethylene derivative compound different from DCE according to the present invention, the hydrocarbon source is subjected to a simplified cracking which produces a mixture of products containing ethylene and other constituents (step a)).

The expression simplified cracking (step a)) is understood to mean, for the purposes of the present invention, all the steps for treating the hydrocarbon source which lead to the formation of a mixture of products containing ethylene and other constituents which will be separated into the fractions A, B and C in step b) of the process according to the invention.

Such a cracking may be carried out according to any known technique as long as it allows the production of a mixture of products containing ethylene and other constituents. Advantageously, the cracking comprises a first cracking step of pyrolysis (that is to say a conversion under the action of heat) of the hydrocarbon source in the presence or absence of third compounds such as water, oxygen, a sulphur derivative and/or a catalyst. This first cracking step of pyrolysis is advantageously carried out in at least one cracking furnace to give rise to the formation of a mixture of cracking products.

This mixture of cracking products advantageously comprises hydrogen, carbon monoxide, carbon dioxide, nitrogen, oxygen, hydrogen sulphide, organic compounds comprising at least one carbon atom, and water.

First cracking step of pyrolysis is preferably carried out in at least two cracking furnaces and particularly preferably in at least three cracking furnaces. First cracking step of pyrolysis is preferably carried out in at most five cracking furnaces and particularly preferably in at most four cracking furnaces. With a more particular advantage, an additional cracking furnace is available to replace one of the furnaces in service when that furnace must undergo a decoking operation.

In a more particularly preferred manner, first cracking step of pyrolysis is carried out in three cracking furnaces. In a most particularly preferred manner, first cracking step of pyrolysis is carried out in three different cracking furnaces, the mixtures of cracking products derived from each of them being gathered together. With a more particular advantage, a fourth cracking furnace is available to replace one of the three furnaces in service when that furnace must undergo a decoking operation.

It is therefore particularly advantageous to carry out first cracking step of pyrolysis in three different cracking furnaces, the mixtures of cracking products derived from each of them being gathered together afterwards and to make a fourth cracking furnace available to replace one of the three furnaces in service.

After this first cracking step of pyrolysis, said mixture of cracking products is subjected to a series of treatment steps making it possible to obtain a mixture of products containing ethylene and other constituents which is advantageously composed of the following steps : thermal recovery of the heat of the cracked gases, optionally organic quenching (optionally including heat recovery across a network of exchangers with intermediate liquids), aqueous quenching, compressing and drying of the gases, and also removing most of the carbon dioxide and most of the sulphur compounds that are present or added (for example, by means of an alkaline wash), optionally hydrogenating undesirable derivatives such as, for example, acetylene and optionally eliminating some of the hydrogen and/or methane, for example via a PSA (pressure swing adsorption) process or via a membrane process.

Advantageously, in the process according to the invention, the mixture of products containing ethylene and other constituents derived from step a) comprises hydrogen, methane, compounds comprising from 2 to 7 carbon atoms, carbon monoxide, nitrogen and oxygen. Hydrogen, methane and compounds comprising from 2 to 7 carbon atoms other than acetylene are preferably present in an amount of at least 200 ppm by volume relative to the total volume of said mixture of products. Carbon monoxide, nitrogen, oxygen and acetylene may be present in an amount of less than 200 ppm by volume or in an amount of at least 200 ppm by volume relative to the total volume of said mixture of products. Compounds containing more than 7 carbon atoms, carbon dioxide, hydrogen sulphide and the other sulphur compounds and also water may also be present in the abovementioned mixture of products in an amount of less than 200 ppm by volume relative to the total volume of said mixture of products.

The compression and drying of the gases may be advantageously performed under particular conditions so that the passage of the compounds comprising at least 6 carbon atoms is minimized. The cooling fluid which may be used is advantageously at a temperature lower than the temperature of the water from an atmospheric cooling tower. The cooling fluid is preferably at a temperature of at least -5°C, more preferably of at least 0°C. The cooling fluid is most preferably iced water.

After step a) defined above, according to step b), the mixture of products containing ethylene and other constituents is separated in one separation step into a fraction containing almost all the ethylene (fraction A) and into a heavy fraction (fraction C).

The expression "one separation step", is understood to mean, for the purpose of the present invention, that one and only one separation is considered.

The separation step is advantageously a fractionation step advantageously involving a fractionation operation.

Examples of fractionation operations are distillation, extractive distillation, liquid-liquid extraction, pervaporation, gas-permeation, adsorption, pressure swing adsorption (PSA), absorption, chromatography, reverse osmosis and molecular filtration. Distillation, gas-permeation, pervaporation and PSA are preferred. Distillation is more preferred.

This separation step therefore more preferably consists in the separation of the mixture of products derived from step a) inside a main column (called column C) into two different fractions, namely fraction A which leaves at the top of column C and fraction C which leaves at the bottom of column C.

Prior to its introduction into column C, the mixture of products derived from step a) may be subjected to a heat conditioning step. The expression heat conditioning step is understood to mean a succession of heat exchanges optimizing the use of energy, for example the gradual cooling of the mixture of products in a train of exchangers first cooled with cooling water, and then with ice-cold water and then with increasingly cooled fluids plus cross exchangers recovering the sensible heat of the streams produced.

The said mixture of products may be introduced into the column C during step b) as a single fraction or as several subfractions. It is preferably introduced as several subfractions.

The main column C is advantageously a column comprising a stripping section and/or a rectifying section. If the two sections are present, the rectifying section preferably surmounts the stripping section.

The column C is advantageously chosen from distillation columns comprising the abovementioned two sections and the columns containing only one of the two sections. Preferably, the column C is a distillation column.

Step b) is therefore preferably a distillation step.

The column C is advantageously provided with the associated auxiliary equipment such as for example at least one reboiler and at least one condenser. Devices allowing intermediate drawing off and an intermediate heat exchange may be added to the main column.

Fraction A containing almost all the ethylene advantageously leaves at the top of column C whereas fraction C enriched with the least volatile compounds advantageously leaves at the bottom of column C.

The abovementioned step b) is advantageously performed at a pressure of at least 8, preferably of at least 10 and in a particularly preferred manner of at least 12 bar. Step b) is advantageously performed at a pressure of at most 45, preferably of at most 40 and in a particularly preferred manner of at most 38 bar.

The temperature at which step b) is performed is advantageously at least -100, preferably at least -90 and in a particularly preferred manner at least -80°C at the top of column C1. It is advantageously at most -30, preferably at most -40 and in a particularly preferred manner at most -50°C at the top of column C1.

Fraction A at the top of the column is advantageously partially condensed to supply the reflux ; the cooling power is advantageously supplied by an external low temperature cycle, an internal low temperature cycle by pressure release of part of the condensed matter or a mixture thereof, preferably by a mixture thereof. An energy recovery by turboexpension of the gas product is optionally used.

The quantities defined below to characterize fraction A and fraction C are those for these fractions leaving the step b).

Fraction A is enriched with compounds which are lighter than ethylene. These compounds are generally methane, nitrogen, oxygen, hydrogen and carbon monoxide. Advantageously, fraction A contains at least 80 %, preferably at least 90 % and in a particularly preferred manner at least 95 % of compounds lighter than ethylene which are contained in the mixture of products subjected to step b).

Fraction A is characterized by a content of compounds containing at least 3 carbon atoms, advantageously less than or equal to 0.1 %, preferably less than or equal to 0.05 % and in a particularly preferred manner less than or equal to 0.01 % by volume relative to the total volume of fraction A.

Fraction A advantageously contains at least 90, preferably at least 95 and more preferably at least 98 % of the ethylene quantity which is contained in the mixture of products subjected to step b).

Fraction C advantageously contains compounds comprising at least 3 carbon atoms. Advantageously, these compounds comprising at least 3 carbon atoms result from the mixture of products containing ethylene and other constituents derived from step a) or are generated by side reactions during step b). Among the compounds comprising at least 3 carbon atoms, there may be mentioned propane, propylene, butanes and their unsaturated derivatives as well as all the saturated or unsaturated heavier compounds.

Fraction C advantageously contains at least 95 %, preferably at least 98 % and particularly preferably at least 99 % of compounds comprising at least 3 carbon atoms contained in the mixture of products subjected to step b).

Fraction C advantageously contains at most 1 %, preferably at most 0.8 % and particularly preferably at most 0.5 % by weight of ethylene relative to the total weight of fraction C.

Fraction C is advantageously enriched in components heavier than ethylene. Preferably, fraction C is burnt as fuel or valorised chemically. More preferably, fraction C is valorised chemically. Fraction C is most preferably subjected to a separation step consisting of separating fraction C, for example by distillation, into two different fractions respectively containing compounds comprising less than 5 carbon atoms for one of the fractions (fraction C1), and compounds comprising at least 5 carbon atoms for the other one (fraction C2). Fraction C1 is then preferably subjected to at least one hydrogenation step before recycling to step a). Fraction C2, particularly enriched with benzene, is particularly preferably conveyed to the manufacture of ethylbenzene. It can therefore be interesting to adapt step b) so that benzene is directed to fraction C in order to maximize its recovery.

In some cases, it can be interesting to isolate ethane in order to valorize it. In these circumstances, the process according to the invention can be adapted so that ethane is directed to fraction C or be isolated as an individual fraction.

In the case ethane is directed to fraction C, ethane can be separated from the heavier hydrocarbons present in fraction C by the use of a further distillation column. Ethane can also be recovered by drawing it off from the side of the distillation column used to isolate fraction C (drawn at the bottom) from the others fraction, or by using a dividing wall column instead of a conventional distillation column when isolating fraction C.

In the case ethane is isolated as an individual fraction, it can be separated from the other fractions during step b).

After having been recovered, ethane can be burnt as fuel or valorized chemically. Ethane is preferably valorized chemically. Ethane is therefore more preferably either recycled to step a) or subjected to an oxydehydrogenation (ODH) as described in patent applications WO2008/000705, WO2008/000702 and WO2008/000693 in order to generate ethylene afterwards subjected to oxychlorination. Ethane is most preferably recycled to step a).

After step b) defined above, according to step c), fraction A is conveyed to the manufacture of at least one ethylene derivative compound.

According to a first embodiment of the process according to the invention, fraction A is advantageoulsy conveyed in one fraction to the manufacture of one ethylene derivative compound.

According to this first embodiment, the process is advantageously such that, after steps a) and b), c) fraction A is conveyed in one fraction to the manufacture of one ethylene derivative compound, preferably to the manufacture of DCE and optionally of any compound derived there from, optionally after having been subjected to an acetylene hydrogenation.

According to a first variant of the first embodiment, the process is advantageously such that, after steps a) and b),
c) fraction A is conveyed in one fraction to the manufacture of DCE, optionally after having been subjected to an acetylene hydrogenation, in a chlorination reactor in which most of the ethylene present in fraction A is converted to DCE by reaction with molecular chlorine ;
d) the DCE obtained is separated from the stream of products derived from the chlorination reactor ;
e) the separated DCE is subjected to a DCE cracking step thus producing VC and hydrogen chloride ; and
f) the VC and hydrogen chloride obtained are separated from the stream of products derived from the DCE cracking step.

The chlorination reaction (usually called direct chlorination) is advantageously carried out in a liquid phase (preferably mainly DCE) containing a dissolved catalyst such as FeCl₃ or another Lewis acid. It is possible to advantageously combine this catalyst with cocatalysts such as alkali metal chlorides. A pair which has given good results is the complex of FeCl₃ with LiCl (lithium tetrachloroferrate - as described in Patent Application NL 6901398).

The amounts of FeCl₃ advantageously used are around 1 to 30 g of FeCl₃ per kg of liquid stock. The molar ratio of FeCl₃ to LiCl is advantageously of the order of 0.5 to 2.

In addition, the chlorination reaction is preferably performed in a chlorinated organic liquid medium. More preferably, this chlorinated organic liquid medium, also called liquid stock, mainly consists of DCE.

The chlorination reaction according to the invention is advantageously performed at temperatures between 30 and 150°C. Good results were obtained regardless of the pressure both at a temperature below the boiling point (chlorination process under subcooled conditions) and at the boiling point itself (process for chlorination at boiling point).

When the chlorination process according to the invention is a chlorination process under subcooled conditions, it gave good results by operating at a temperature which was advantageously greater than or equal to 50°C and preferably greater than or equal to 60°C, but advantageously less than or equal to 80°C and preferably less than or equal to 70°C, and with a pressure in the gaseous phase advantageously greater than or equal to 1 and preferably greater than or equal to 1.1 bar absolute, but advantageously less than or equal to 20, preferably less than or equal to 10 and particularly preferably less than or equal to 6 bar absolute.

A process for chlorination at boiling point may be preferred to usefully recover the heat of reaction. In this case, the reaction advantageously takes place at a temperature greater than or equal to 60°C, preferably greater than or equal to 70°C and particularly preferably greater than or equal to 85°C, but advantageously less than or equal to 150°C and preferably less than or equal to 135°C, and with a pressure in the gaseous phase advantageously greater than or equal to 0.2, preferably greater than or equal to 0.5, particularly preferably greater than or equal to 1.1 and more particularly preferably greater than or equal to 1.3 bar absolute, but advantageously less than or equal to 10 and preferably less than or equal to 6 bar absolute.

The chlorination process may also be a hybrid loop-cooled process for chlorination at boiling point. The expression "hybrid loop-cooled process for chlorination at boiling point" is understood to mean a process in which cooling of the reaction medium is carried out, for example, by means of an exchanger immersed in the reaction medium or by a loop circulating in an exchanger, while producing in the gaseous phase at least the amount of DCE formed. Advantageously, the reaction temperature and pressure are adjusted for the DCE produced to leave in the gaseous phase and for the remainder of the heat from the reaction medium to be removed by means of the exchange surface area.

Fraction submitted to the chlorination and also the molecular chlorine (itself pure or diluted) may be introduced, together or separately, into the reaction medium by any known device. A separate introduction of the fraction submitted to the chlorination may be advantageous in order to increase its partial pressure and facilitate its dissolution which often constitutes a limiting step of the process.

The molecular chlorine is added in a sufficient amount to convert most of the ethylene and without requiring the addition of an excess of unconverted chlorine. The chlorine/ethylene ratio used is preferably between 1.2 and 0.8 and particularly preferably between 1.05 and 0.95 mol/mol.

The chlorinated products obtained contain mainly DCE and also small amounts of by-products such as 1,1,2-trichloroethane or small amounts of ethane or methane chlorination products.

The separation of the DCE obtained from the stream of products derived from the chlorination reactor is carried out according to known modes and in general makes it possible to exploit the heat of the chlorination reaction. It is then preferably carried out by condensation and gas/liquid separation.

The unconverted products (methane, ethane, carbon monoxide, nitrogen, oxygen and hydrogen) are then advantageously subjected to an easier separation than what would have been necessary to separate pure ethylene starting from the initial mixture.

Hydrogen in particular can be extracted from the unconverted products and be valorized as for example for the hydrogenation of working solution in hydrogen peroxide manufacture or for the direct synthesis of hydrogen peroxide.

The conditions under which the DCE cracking step may be carried out are known to persons skilled in the art. The DCE cracking can be performed in the presence or in the absence of third compounds among which can be cited the catalysts ; the DCE cracking is in this case a catalytic DCE cracking. The DCE cracking is however preferably performed in the absence of third compounds and under the action of heat only ; the DCE cracking is in this case often called pyrolysis.

This pyrolysis is advantageously obtained by a reaction in the gaseous phase in a tubular oven. The usual pyrolysis temperatures are between 400 and 600°C with a preference for the range between 480°C and 540°C. The residence time is advantageously between 1 and 60 seconds with a preference for the range from 5 to 25 seconds. The rate of conversion of the DCE is advantageously limited to 45 to 75 % in order to limit the formation of by-products and the fouling of the tubes of the oven.

The separation of the VC and hydrogen chloride obtained from the stream of products derived from the pyrolysis is carried out according to known modes, using any known device, in order to collect the purified VC and the hydrogen chloride. Following purification, the unconverted DCE is advantageously conveyed to the pyrolysis oven.

According to the first variant of the first embodiment, VC is afterwards preferably polymerized to produce PVC.

The manufacture of PVC may be a mass, solution or aqueous dispersion polymerization process, preferably it is an aqueous dispersion polymerization process.

The expression aqueous dispersion polymerization is understood to mean free radical polymerization in aqueous suspension as well as free radical polymerization in aqueous emulsion and polymerization in aqueous microsuspension.

The expression free radical polymerization in aqueous suspension is understood to mean any free radical polymerization process performed in aqueous medium in the presence of dispersing agents and oil-soluble free radical initiators.

The expression free radical polymerization in aqueous emulsion is understood to mean any free radical polymerization process performed in aqueous medium in the presence of emulsifying agents and water-soluble free radical initiators.

The expression aqueous microsuspension polymerization, also called polymerization in homogenized aqueous dispersion, is understood to mean any free radical polymerization process in which oil-soluble initiators are used and an emulsion of droplets of monomers is prepared by virtue of a powerful mechanical stirring and the presence of emulsifying agents.

After separation, hydrogen chloride may be used for any purpose. It can for example be conveyed to the synthesis of compounds like calcium chloride, chloro(s) alcohol(s) among which chloro(s) propanol(s) by reaction with 1,2-propanediol, 1,3-propanediol or 1,2,3-propanetriol (glycerin or glycerol leading to the synthesis of epichlorhydrin), chloro(s) alcane(s) among which chloro(s) methane by reaction with methanol, aqueous hydrochloric acid, ferric chloride, aluminium chloride, chlorosilanes, titanium chloride, zinc chloride, other inorganic chlorides like ammonium chloride but also to oxychlorination processes for example of aromatic compounds, hydrochlorination of alkynes (for example hydrochlorination of acetylene into VC) or of alkenes or be oxidized to molecular chlorine.

After separation according to step f) of the first variant of the first embodiment of the process according to the invention, g) hydrogen chloride is preferably subjected to an oxidation into molecular chlorine which is afterwards more preferably recycled to the chlorination reactor.

A particular preferred process is therefore such that, after steps a) and b),
c) fraction A is conveyed in one fraction to the manufacture of DCE, optionally after having been subjected to an acetylene hydrogenation, in a chlorination reactor in which most of the ethylene present in fraction A is converted to DCE by reaction with molecular chlorine ;
d) the DCE obtained is separated from the stream of products derived from the chlorination reactor ;
e) the separated DCE is subjected to a DCE cracking step thus producing VC and hydrogen chloride ;
f) the VC and hydrogen chloride obtained are separated from the stream of products derived from the DCE cracking step ; and
g) hydrogen chloride is subjected to an oxidation into molecular chlorine which is afterwards recycled to the chlorination reactor.

The oxidation of the separated hydrogen chloride into molecular chlorine can be made according to any known process.

Among those known processes may be cited the electrolysis of hydrochloric acid, the catalytic oxidation processes of hydrogen chloride by oxygen like the KEL chlorine process called Kellogg (using concentrated sulfuric acid and nitrosylsulfuric acid as catalyst), the Shell-Deacon process (using a mixture of copper(II) chloride and other metallic chlorides on a silicate carrier as catalyst) and modified Deacon processes like the Mitsui-Toatsu (MT-Chlorine) process (using a chromium(III) oxide on a silicate carrier as catalyst) as well as the oxidation of hydrogen chloride by nitric acid.

Catalytic oxidation of hydrogen chloride by oxygen is preferred for the process according to the invention. This oxidation is advantageously performed with a gas containing oxygen.

As the gas containing oxygen, molecular oxygen or air can be used. Oxygen may be produced by usual industrial methods such as pressure-swing method of air or deep-cooling separation of air.

While the theoretical molar amount of oxygen necessary for oxidizing one mole of hydrogen chloride is 0.25 mole, it is preferable to use oxygen in an amount exceeding the theoretical amount, and more preferably, 0.25 to 2 moles of oxygen is used per one mole of hydrogen chloride.

The catalyst used in the oxidation reaction according to the present invention may be any known catalyst that is used in the production of chlorine through the oxidation of hydrogen chloride.

Examples of catalysts are copper-based catalysts as in the Deacon process, chromium oxide, ruthenium oxide or mixture of ruthenium oxide and titanium oxide. Deacon catalysts comprises advantageously copper chloride, potassium chloride and various kinds of compounds a third components.

The shape of the catalyst may be any of conventionally used shapes such as a spherical particle, a cylindrical pellet, an extruded form, a ring form, a honeycomb form, or a granule having a suitable size which is produced by milling of a molded material followed by sieving. The size of the catalyst is preferably 10 mm or less. Although the lower limit of the size of the catalyst may not be limited, the size of the catalyst is advantageously at least 0.1 mm. Herein, the size of the catalyst means a diameter of a sphere in the case of the spherical particle, a diameter of a cross section in the case of the cylindrical pellet or the largest size of the cross section in the case of other forms.

It can be interesting to divide the gas containing oxygen into portions and introduced it in at least two reaction zones.

The oxidation reaction is advantageously carried out in at least two reaction zones each comprising a catalyst-packed layer, preferable arranged in series.

The reaction pressure is advantageously from 0.1 to 5 MPa. The reaction temperature is advantageously from 200 to 650°C, more preferably from 200 to 500°C.

The reactors are advantageously tubular reactors, the inner diameter of which are preferably from 10 to 50 mm, more preferably from 10 to 40 mm.

The molecular chlorine is more preferably recycled to the chlorination reactor. The recycling can be made according to any known process. The molecular chlorine is advantageously first dried and then put at the required pressure for entering chlorination. The drying is advantageously performed either by a compression with a condensation at the outlet or with the use of a column with sulfuric acid or with an adsorbent compatible with chlorine, preferably with a column with sulfuric acid.

According to a second variant of the first embodiment, the process is preferably such that, after steps a) and b),
c) fraction A is conveyed in one fraction to the manufacture of DCE, optionally after having been subjected to an acetylene hydrogenation, in a chlorination reactor in which at most 90 % of the ethylene present in fraction A is converted to DCE by reaction with molecular chlorine ;
d) the DCE formed in the chlorination reactor is optionally isolated from the stream of products derived from the chlorination reactor ;
e) the stream of products derived from the chlorination reactor, from which the DCE has optionally been extracted, is conveyed to an oxychlorination reactor in which the majority of the balance of ethylene is converted to DCE, after optionally having subjected the latter to an absorption/desorption step e'), during which the DCE formed in the chlorination reactor is optionally extracted if it has not previously been extracted ; and
f) the DCE formed in the oxychlorination reactor is isolated from the stream of products derived from the oxychlorination reactor and is optionally added to the DCE formed in the chlorination reactor.

According to this second variant of the first embodiment, DCE is advantageously further subjected to a DCE cracking step to produce VC and VC is afterwards preferably polymerized to produce PVC.

Reference is made to the first variant of the first embodiment for the details about the chlorination reaction in the particular case of the second variant of the first embodiment except for the flow of chlorine detailed here after.

The flow of chlorine is such that advantageously at least 10 %, preferably at least 20 % and particularly preferably at least 30 % of the ethylene is converted to DCE. The flow of chlorine is such that advantageously at most 90 %, preferably at most 80 % and particularly preferably at most 70 % of the ethylene is converted to DCE.

According to step d) of the second variant of the first embodiment, the DCE formed in the chlorination reactor is optionally isolated from the stream of products derived from the chlorination reactor. In certain cases it may be advantageous not to isolate the DCE formed in the chlorination reactor from the stream of products derived from the chlorination reactor. Preferably however, the DCE formed in the chlorination reactor is isolated from the stream of products derived from the chlorination reactor.

When it takes place, the separation of the DCE obtained from the stream of products derived from the chlorination reactor is carried out according to known methods and in general makes it possible to exploit the heat of the chlorination reaction. It is then preferably carried out by condensation and gas/liquid separation.

According to step e) of the second variant of the first embodiment, the stream of products derived from the chlorination reactor, from which the DCE has optionally been extracted, is conveyed to an oxychlorination reactor in which the majority of the balance of ethylene is converted to DCE, after optionally having subjected the latter to an absorption/desorption step e'), during which the DCE formed in the chlorination reactor is optionally extracted if it has not previously been extracted.

The oxychlorination reaction is advantageously performed in the presence of a catalyst comprising active elements including copper deposited on an inert support. The inert support is advantageously chosen from alumina, silica gels, mixed oxides, clays and other supports of natural origin. Alumina constitutes a preferred inert support.

Catalysts comprising active elements which are advantageously at least two in number, one of which is copper, are preferred. Among the active elements other than copper, mention may be made of alkali metals, alkaline-earth metals, rare-earth metals and metals from the group consisting of ruthenium, rhodium, palladium, osmium, iridium, platinum and gold. The catalysts containing the following active elements are particularly advantageous : copper/magnesium/potassium, copper/magnesium/sodium; copper/magnesium/lithium, copper/magnesium/caesium, copper/magnesium/sodium/lithium, copper/magnesium/potassium/lithium and copper/magnesium/caesium/lithium, copper/magnesium/sodium/potassium, copper/magnesium/sodium/caesium and copper/magnesium/potassium/caesium. The catalysts described in Patent Applications EP-A 255 156, EP-A 494 474, EP-A-657 212 and EP-A 657 213, incorporated by reference, are most particularly preferred.

The copper content, calculated in metal form, is advantageously between 30 and 90 g/kg, preferably between 40 and 80 g/kg and particularly preferably between 50 and 70 g/kg of catalyst.

The magnesium content, calculated in metal form, is advantageously between 10 and 30 g/kg, preferably between 12 and 25 g/kg and particularly preferably between 15 and 20 g/kg of catalyst.

The alkali metal content, calculated in metal form, is advantageously between 0.1 and 30 g/kg, preferably between 0.5 and 20 g/kg and particularly preferably between 1 and 15 g/kg of catalyst.

The Cu:Mg:alkali metal(s) atomic ratios are advantageously 1:0.1-2:0.05-2, preferably 1:0.2-1.5:0.1-1.5 and particularly preferably 1:0.5-1:0.15-1.

Catalysts having a specific surface area, measured according to the BET method with nitrogen that is advantageously between 25 m²/g and 300 m²/g, preferably between 50 and 200 m²/g and particularly preferably between 75 and 175 m²/g, are particularly advantageous.

The catalyst may be used in a fixed bed or in a fluidized bed. This second option is preferred. The oxychlorination process is operated under the range of the conditions usually recommended for this reaction. The temperature is advantageously between 150 and 300°C, preferably between 200 and 275°C and most preferably from 215 to 255°C. The pressure is advantageously above atmospheric pressure. Values of between 2 and 10 bar absolute gave good results. The range between 4 and 7 bar absolute is preferred. This pressure may be usefully adjusted in order to attain an optimum residence time in the reactor and to maintain a constant rate of passage for various operating speeds. The usual residence times range from 1 to 60 s and preferably from 10 to 40 s.

The source of oxygen for this oxychlorination may be air, pure oxygen or a mixture thereof, preferably pure oxygen. The latter solution, which allows easy recycling of the unconverted reactants, is preferred.

The reactants may be introduced into the bed by any known device. It is generally advantageous to introduce the oxygen separately from the other reactants for safety reasons. These safety reasons also require the gaseous mixture leaving the reactor or recycled thereto to be kept outside the limits of inflammability at the pressures and temperatures in question. It is preferable to maintain a so-called rich mixture, that is to say containing too little oxygen relative to the fuel to ignite. In this regard, the abundant presence (> 2 vol %, preferably > 5 vol %) of hydrogen would constitute a disadvantage given the wide range of inflammability of this compound.

The hydrogen chloride/oxygen ratio used is advantageously between 3 and 6 mol/mol. The ethylene/hydrogen chloride ratio is advantageously between 0.4 and 0.6 mol/mol.

The chlorinated products obtained contain mainly DCE and also small amounts of by-products such as 1,1,2-trichloroethane.

In certain cases, it may be advantageous, before entering into the oxychlorination reactor, to subject the stream of products derived from the chlorination reactor, from which the DCE has optionally been extracted, to the absorption/desorption step e'), during which the DCE formed in the chlorination reactor is optionally extracted if it has not previously been extracted.

The expression "step e'), during which the DCE formed in the chlorination reactor is optionally extracted if it has not previously been extracted" is understood to mean that the DCE formed in the chlorination reactor may be extracted during step e') if this step takes place and if it has not previously been extracted. Preferably, the DCE formed in the chlorination reactor is extracted during step e') if this step takes place and if it has not previously been extracted.

Thus, the stream of products derived from the chlorination reactor, from which the DCE has optionally been extracted, (known hereinafter as chlorination stream) is advantageously subjected to an absorption step and to a desorption step in which said stream is preferably brought into contact with a washing agent containing a solvent.

The expression "washing agent containing a solvent" or more simply "washing agent" is understood to mean a composition in which the solvent is present in the liquid state.

The washing agent that can be used according to the present invention therefore advantageously contains a solvent in the liquid state. The presence, in said washing agent, of other compounds is not at all excluded from the scope of the invention. However, it is preferred that the washing agent contain at least 50 % by volume of the solvent, more particularly at least 65 % by volume and most particularly preferably at least 70 % by volume.

The solvent is advantageously chosen among the alcohols, glycols, polyols, ethers, mixtures of glycol(s) and ether(s), mineral oils as well as DCE. The solvent is preferably chosen among the alcohols, the mineral oils and DCE and more preferably among azeotropic ethanol (aqueous ethanol with advantageously at least 70, preferably at least 80 and more preferably at least 85 % by volume of ethanol) and DCE. The solvent is most preferably DCE.

The washing agent used for the absorption step may be composed of fresh washing agent of any origin, for example crude azeotropic ethanol or crude DCE exiting the chlorination unit, crude DCE exiting the oxychlorination unit or a mixture of the two which has not been purified. It may also be composed of said DCE that has been previously purified or all or part of the washing agent recovered during the desorption step explained below optionally containing the DCE formed in the chlorination reactor and extracted in the desorption step, after an optional treatment making it possible to reduce the concentration, in the DCE, of the compounds that are heavier than ethane, as explained below, optionally with the addition of fresh washing agent.

Preferably, the washing agent used for the absorption step is composed of all or part of the washing agent recovered during the desorption step optionally containing the DCE formed in the chlorination reactor and extracted in the desorption step, after the abovementioned optional treatment, optionally with the addition of fresh washing agent. In the case where the DCE formed in the chlorination reaction is isolated from the stream of products derived from the chlorination reactor at the chlorination outlet, in a particularly preferred manner, the washing agent used for the absorption step is composed of all or part of the washing agent recovered during the desorption step, after the aforementioned optional treatment, with the addition of fresh washing agent (to compensate for losses of washing agent during the absorption and desorption steps).

The abovementioned optional treatment making it possible to reduce the concentration, in the washing agent, of the compounds that are heavier than ethane, preferably of the compounds comprising at least 3 carbon atoms, may be a step of desorbing the compounds that are heavier than ethane and lighter than the washing agent or a step of distilling the washing agent. Preferably, it consists of desorbing the compounds that are heavier than ethane and lighter than the washing agent. Preferably, this treatment of the washing agent takes place.

An essential advantage of the most preferred case when DCE is the washing agent, lies in the fact that the presence of this DCE is not at all troublesome, as it is the compound mainly formed during the oxychlorination or chlorination.

The ratio between the respective throughputs of washing agent and the chlorination stream is not critical and can vary to a large extent. It is in practice limited only by the cost of regenerating the washing agent. In general, the throughput of washing agent is at least 1, preferably at least 5 and particularly preferably at least 10 tonnes per tonne of chlorination stream. In general, the throughput of washing agent is at most 100, preferably at most 50 and particularly preferably at most 25 tonnes per tonne of the ethylene and ethane mixture to be extracted from the chlorination stream.

The absorption step is advantageously carried out by means of an absorber such as, for example, a climbing film or falling film absorber or an absorption column chosen from plate columns, columns with random packing, columns with structured packing, columns combining one or more of the aforementioned internals and spray columns. The absorption step is preferably carried out by means of an absorption column and particularly preferably by means of a plate absorption column.

The absorption column is advantageously equipped with associated accessories such as, for example, at least one condenser or chiller that is internal or external to the column.

The abovementioned absorption step is advantageously carried out at a pressure of at least 15, preferably of at least 20 and particularly preferably of at least 25 bar absolute. The absorption step is advantageously carried out at a pressure of at most 40, preferably at most 35 and particularly preferably at most 30 bar absolute.

The temperature at which the absorption step is carried out is advantageously at least -10, preferably at least 0 and particularly preferably at least 10°C at the top of the absorber or absorption column. It is advantageously at most 60, preferably at most 50 and particularly preferably at most 40°C at the top of the absorber or absorption column.

The temperature at the bottom of the absorber or absorption column is at least 0, preferably at least 10 and particularly preferably at least 20°C. It is advantageously at most 70, preferably at most 60 and particularly preferably at most 50°C.

The stream resulting from the absorption step, which is the chlorination stream purified of compounds that are lighter than ethylene and enriched in washing agent is advantageously subjected to the desorption step.

The washing agent recovered after the desorption step optionally containing the DCE formed in the chlorination reactor then extracted may be removed, completely or partly conveyed to the oxychlorination sector where the DCE comes together with the DCE formed in the oxychlorination reactor, or completely or partly reconveyed to the absorption step, optionally after the abovementioned treatment, with the optional addition of fresh washing agent. Preferably, the washing agent recovered after the desorption step is completely or partly reconveyed to the absorption step, after the abovementioned optional treatment, with optional addition of fresh washing agent, or to the oxychlorination sector. In the case where the DCE formed in the chlorination reactor is isolated from the stream of products derived from the chlorination reactor at the chlorination outlet, in a particularly preferred manner, the washing agent recovered after the desorption step is completely or partly reconveyed to the absorption step, after the abovementioned optional treatment, with addition of fresh washing agent.

The desorption step is advantageously carried out by means of a desorber such as, for example, a climbing film or falling film desorber, a reboiler or a desorption column chosen from plate columns, columns with random packing, columns with structured packing, columns combining one or more of the aforementioned internals and spray columns. The desorption can also be performed by direct injection of vapour in order to collect DCE. The desorption step is preferably carried out by means of a desorption column and particularly preferably by means of a plate desorption column.

The desorption column is advantageously equipped with associated accessories such as, for example, at least one condenser or one chiller that is internal or external to the column and at least one reboiler.

The desorption pressure is advantageously chosen so that the content of compounds having at least 3 carbon atoms in the desorbed gas is less than 100 ppm, preferably less than or equal to 50 ppm and particularly preferably less than or equal to 20 ppm by volume.

The abovementioned desorption step is advantageously carried out at a pressure of at least 1, preferably at least 2 and particularly preferably at least 3 bar absolute. The desorption step is advantageously carried out at a pressure of at most 20, preferably at most 15 and particularly preferably at most 10 bar absolute.

The temperature at which the desorption step is carried out is advantageously at least -10, preferably at least 0 and particularly preferably at least 10°C at the top of the desorber or desorption column. It is advantageously at most 60, preferably at most 50 and particularly preferably at most 45°C at the top of the desorber or desorption column.

The temperature at the bottom of the desorber or desorption column is at least 60, preferably at least 80 and particularly preferably at least 100°C. It is advantageously at most 200, preferably at most 160 and particularly preferably at most 150°C.

A most particular preference is attached to the case where the absorption step is carried out in an absorption column and the desorption step in a desorption column.

The hydrogen recovered following the absorption step is advantageously developed as a fuel or as a reactant, optionally after a purification step. Thus, the hydrogen may be developed as a fuel in the DCE cracking step. It may also be developed as a reactant for a hydrogenation reaction for example.

According to step f) of the second variant of the first embodiment, the DCE formed in the oxychlorination reactor is isolated from the stream of products derived from the oxychlorination reactor and is optionally added to the DCE formed in the chlorination reactor.

The separation of the DCE obtained from the stream of products derived from the oxychlorination reactor is carried out according to known methods. It is preferably carried out first by condensation. The heat of the oxychlorination reactor is generally recovered in the vapour state which may be used for the separations or for any other use.

After exiting from the oxychlorination reactor, the stream of products derived from the reactor is also advantageously washed to recover the unconverted HCl. This washing operation is advantageously an alkaline washing step. It is preferably followed by a gas/liquid separation step which makes it possible to recover the DCE formed in liquid form and finally to dry the DCE.

The expression "is optionally added to the DCE formed in the chlorination reactor" is understood to mean that if the DCE formed in the chlorination reactor is isolated from the stream of products derived from this reactor, on exiting the chlorination reactor or after step e'), the DCE formed in the oxychlorination reactor may or may not be added thereto. Preferably, it is added thereto. If on the other hand, this first DCE is not isolated, the DCE isolated from the stream of products derived from the oxychlorination reactor is advantageously the only stream of DCE recovered. Another alternative is advantageously to mix the DCE isolated from the stream of products derived from the oxychlorination reactor with a part of the DCE isolated from the stream of products derived from the chlorination reactor and to send the other part of this latter directly to the DCE cracking step.

Reference is made to the first variant of the first embodiment for more details about the DCE cracking step and about the separation of the VC obtained from the stream of products derived from the DCE cracking step.

According to this second variant of the first embodiment, VC is afterwards preferably polymerized to produce PVC. Reference is made to the first variant of the first embodiment for more details about the manufacture of PVC.

According to a second embodiment of the process according to the invention, fraction A is advantageoulsy divided into at least two fractions of the same composition or of different composition, preferably into fraction A1 and fraction A2 of the same composition or of different composition.

According to this second embodiment, the process is advantageously such that, after steps a) and b), c) fraction A is divided into at least two fractions, preferably into fraction A1 and fraction A2, of the same composition or of different composition before being conveyed to the manufacture of at least one ethylene derivative compound.

The separation of fraction A into at least two fractions, preferably into fraction A1 and fraction A2, is advantageously operated by divided fraction A into several, preferably two, fractions of the same composition or of different composition by means of any known means.

The case when fraction A is divided into at least two, preferably into fraction A1 and fraction A2, of the same composition is particularly interesting when the mixture of products containing ethylene and other constituents leaving step a) can simply be divided, preferably by two, preferably when the mixture of products leaving step a) is poor in hydrogen and/or rich in compounds reacting with hydrogen during hydrogenation steps or when step a8) is performed.

The case when fraction A is divided into at least two fractions, preferably into fraction A1 and fraction A2, of different composition is particularly interesting when fractions of different composition are required for step c). Fraction A is therefore advantageously divided into at least two fractions, preferably into fraction A1 and fraction A2, of different composition so that each fraction can be conveyed to the respective manufacture of ethylene derivative compound.

The division of fraction A into at least two fractions, preferably into fraction A1 and fraction A2, can be made by any known means. Preferably, fraction A is cooled down by indirect cooling in a heat exchanger where fraction A2 is vaporized after expansion to a suitable pressure and overcooled by indirect contact in an heat exchanger cooled with a suitable cooling media up to a defined lowering of its temperature. The liquid vapor is the preferably divided to produce the vapor fraction A1 and the liquid fraction A2. The temperature lowering is advantageously greater than 5, preferably greater than 7 and more preferably greater than 8°C. The temperature lowering is advantageously lower than 30, preferably lower than 25 and more preferably lower than 22°C.

Fraction A1 advantageously contains more than 10, preferably more than 20 and more preferably more than 25 % the ethylene quantity which is contained in fraction A. Fraction A1 advantageously contains less than 90, preferably less than 80 and more preferably less than 75 % the ethylene quantity which is contained in fraction A.

Fraction A1 advantageously contains more than 80, preferably more than 85 and more preferably more than 90 % the hydrogen quantity which is contained in fraction A.

Fraction A1 advantageously contains more than 70, preferably more than 75 and more preferably more than 80 % the methane quantity which is contained in fraction A.

Fraction A1 advantageously contains less than 40, preferably less than 30 and more preferably less than 25 % of the ethane quantity which is contained in fraction A.

According to a first variant of the second embodiment, the process is advantageously such that, after steps a) and b),
c) fraction A is divided into fraction A1 and fraction A2 of the same composition or of different composition, fraction A1 and fraction A2 being conveyed to the manufacture of DCE and optionally of any compound derived there from, optionally after having been subjected to an acetylene hydrogenation.

The process according to this first variant of the second embodiment is preferably such that, after steps a), b) and c),
d) fraction A1 is conveyed to a chlorination reactor and fraction A2 to an oxychlorination reactor, optionally after having been subjected to an acetylene hydrogenation, in which reactors most of the ethylene present in fractions A1 and A2 is converted to DCE ; and
e) the DCE obtained is separated from the streams of products derived from the chlorination and oxychlorination reactors.

According to a second variant of the second embodiment, the process is advantageously such that, after steps a) and b),
c) fraction A is divided into fraction A1 and fraction A2 of the same composition or of different composition, one of which being conveyed to the manufacture of DCE and optionally of any compound derived there from, optionally after having been subjected to an acetylene hydrogenation, while the other is conveyed to the manufacture of at least one ethylene derivative compound manufactured directly starting with ethylene which is different from DCE and optionally of any compound derived there from.

The process according to this second variant of the second embodiment is preferably such that, after steps a) and b),
c) fraction A is divided into fraction A1 and fraction A2 of the same composition or of different composition, fraction A1 being conveyed to the manufacture of DCE and optionally of any compound derived there from, optionally after having been subjected to an acetylene hydrogenation, while fraction A2 is conveyed to the manufacture of at least one ethylene derivative compound manufactured directly starting with ethylene which is different from DCE and optionally of any compound derived there from.

The three variants detailed for the first embodiment of the process according to the invention in order to obtain DCE and afterwards VC and PVC from fraction A apply also for the second variant of the second embodiment of the process according to the invention in order to obtain DCE and afterwards VC and PVC from fraction A1.

According to the second variant of the second embodiment, fraction A2 is advantageously conveyed to the manufacture of at least one ethylene derivative compound manufactured directly starting with ethylene which is different from DCE and optionally of any compound derived there from.

As examples of ethylene derivative compounds manufactured directly starting with ethylene which are different from DCE which can be manufactured from fraction B may be cited among others, ethylene oxide, linear alpha-olefines, linear primary alcohols, homopolymers and copolymers of ethylene, ethylbenzene, vinyl acetate, acetaldehyde, ethyl alcohol and propionaldehyde.

As examples of the optional compound derived there from, may be cited among others, glycols manufactured from ethylene oxide, styrene manufactured from ethylbenzene and polymers of styrene derived from styrene.

Fraction A2 can be conveyed to the manufacture of one or of several ethylene derivative compounds manufactured directly starting with ethylene which are different from DCE.

In order to be sent to the manufacture of several ethylene derivative compounds manufactured directly starting with ethylene which are different from DCE, fraction A2 is advantageously divided into as many fractions of the same composition as necessary.

Preferably, fraction A2 is conveyed to the manufacture of one ethylene derivative compound manufactured directly starting with ethylene which is different from DCE.

Fraction A2 is more preferably conveyed to the manufacture of ethylbenzene and most preferably to the manufacture of ethylbenzene itself conveyed to the manufacture of styrene afterwards polymerized in order to obtain polymers of styrene.

According to the second embodiment, DCE is more preferably further subjected to a DCE cracking step to produce VC and VC is afterwards most preferably polymerized to produce PVC.

The DCE separated from the streams of products derived from the chlorination reactor can be mixed or not with the DCE separated from the streams of products derived from the oxychlorination reactor before the DCE cracking step. When both DCE are mixed, they can be mixed totally or partially. A preferred case is when DCE isolated from the stream of products derived from the oxychlorination reactor is mixed with a part of the DCE isolated from the stream of products derived from the chlorination reactor and the other part of this latter is sent directly to the DCE cracking step.

Reference is made to the first variant of the first embodiment for the details about the chlorination reaction and the separation of the DCE obtained from the stream of products derived from the chlorination reactor. Reference is also made to the same first variant for the details about the DCE cracking step and the separation of the VC obtained from the stream of products derived from the DCE cracking step. Reference is made to the second variant of the first embodiment for the details about the oxychlorination reaction and the separation of the DCE obtained from the stream of products derived from the oxychlorination reactor.

According to this second embodiment, VC is afterwards preferably polymerized to produce PVC. Reference is made to the first variant of the first embodiment for more details about the manufacture of PVC.

An advantage of the process according to the invention is that it allows a separation step b) which is simplified compared with corresponding separation steps described in the previous patent applications, allowing a lower energy demand.

An advantage of the second variant of the second embodiment of the process according to the invention is that it allows the integration of the DCE manufacture with the manufacture of at least one ethylene derivative compound different from DCE.

This integration allows a reduction of the total cost thanks to the sharing of the costs linked to the common steps.

In the particular case of an integration of DCE/VC/PVC manufacture with ethylbenzene/styrene/polystyrene manufacture, the process allows further the valorization of the fraction enriched in benzene (fraction C2 here above).

An advantage of the process according to the invention is that it makes it possible to have, on the same industrial site, a completely integrated process.

## Claims

1. Process for the manufacture of at least one ethylene derivative compound starting with a hydrocarbon source according to which :
a) the hydrocarbon source is subjected to a simplified cracking which produces a mixture of products containing ethylene and other constituents ;
b) the said mixture of products is separated in one separation step into a fraction containing almost all the ethylene (fraction A) and into a heavy fraction (fraction C) ;
c) fraction A is conveyed to the manufacture of at least one ethylene derivative compound.

2. Process according to Claim 1 according to which after steps a) and b),
c) fraction A is conveyed in one fraction to the manufacture of 1,2-dichloroethane and optionally of any compound derived there from, optionally after having been subjected to an acetylene hydrogenation.

3. Process according to Claim 2, according to which after steps a) and b),
c) fraction A is conveyed in one fraction to the manufacture of 1,2-dichloroethane, optionally after having been subjected to an acetylene hydrogenation, in a chlorination reactor in which most of the ethylene present in fraction A is converted to 1,2-dichloroethane by reaction with molecular chlorine ;
d) the 1,2-dichloroethane obtained is separated from the stream of products derived from the chlorination reactor ;
e) the separated 1,2-dichloroethane is subjected to a DCE cracking step thus producing vinyl chloride and hydrogen chloride ;
f) the vinyl chloride and hydrogen chloride obtained are separated from the stream of products derived from a DCE cracking step ; and
g) hydrogen chloride is subjected to an oxidation into molecular chlorine which is afterwards recycled to the chlorination reactor.

4. Process according to Claim 3, according to which vinyl chloride is polymerized to produce polyvinyl chloride.

5. Process according to Claim 2, according to which after steps a) and b),
c) fraction A is conveyed in one fraction to the manufacture of 1,2-dichloroethane, optionally after having been subjected to an acetylene hydrogenation, in a chlorination reactor in which at most 90 % of the ethylene present in fraction A is converted to 1,2-dichloroethane by reaction with molecular chlorine ;
d) the 1,2-dichloroethane formed in the chlorination reactor is optionally isolated from the stream of products derived from the chlorination reactor ;
e) the stream of products derived from the chlorination reactor, from which the 1,2-dichloroethane has optionally been extracted, is conveyed to an oxychlorination reactor in which the majority of the balance of ethylene is converted to 1,2-dichloroethane, after optionally having subjected the latter to an absorption/desorption step e'), during which the 1,2-dichloroethane formed in the chlorination reactor is optionally extracted if it has not previously been extracted ; and
f) the 1,2-dichloroethane formed in the oxychlorination reactor is isolated from the stream of products derived from the oxychlorination reactor and is optionally added to the 1,2-dichloroethane formed in the chlorination reactor.

6. Process according to Claim 5, according to which 1,2-dichloroethane is subjected to a DCE cracking step to produce vinyl chloride and vinyl chloride is afterwards polymerized to produce polyvinyl chloride.

7. Process according to Claim 1 according to which after steps a) and b),
c) fraction A is divided into at least two fractions, preferably into fraction A1 and fraction A2, of the same composition or of different composition before being conveyed to the manufacture of at least one ethylene derivative compound.

8. Process according to Claim 7, according to which after steps a) and b),
c) fraction A is divided into fraction A1 and fraction A2 of the same composition or of different composition, fraction A1 and fraction A2 being conveyed to the manufacture of DCE and optionally of any compound derived there from, optionally after having been subjected to an acetylene hydrogenation.

9. Process according to Claim 7, according to which after steps a) and b),
c) fraction A is divided into fraction A1 and fraction A2 of the same composition or of different composition, one of which being conveyed to the manufacture of DCE and optionally of any compound derived there from, optionally after having been subjected to an acetylene hydrogenation, while the other is conveyed to the manufacture of at least one ethylene derivative compound manufactured directly starting with ethylene which is different from DCE and optionally of any compound derived there from.

10. Process according to any one of Claims 1 to 9, **characterized in that** the hydrocarbon source is chosen from the group consisting of naphtha, gas oil, natural gas liquid, ethane, propane, butane, isobutane and mixtures thereof.

11. Process according to Claim 10, **characterized in that** the hydrocarbon source is chosen from the group consisting of ethane, propane, butane and propane/butane mixtures.

12. Process according to any one of Claims 1 to 11, **characterized in that** fraction A contains at least 90 % of the ethylene quantity which is contained in the mixture of products subjected to step b).

13. Process according to any one of Claims 1 to 12, **characterized in that** fraction C is burnt as fuel or valorised chemically.
